# EUROPEAN PATENT APPLICATION

(11) **EP 0 883 049 A1**
(43) Date of publication of application: **09.12.1998**
(21) Application number: 98109715.7
(22) Date of filing: 28.05.1998
(51) Int. Cl.: G05D 11/13, G01N 33/00

(54) **Olfactometer**

(30) Priority: 05.06.1997 EP 97109079; 23.06.1997 EP 97110247
(71) Applicant: GIVAUDAN-ROURE (INTERNATIONAL) S.A., 1214 Vernier, Genève (CH)
(72) Inventor: Gygax, Hansruedi, 7310 Bad Ragaz (CH); Montagner, Christian, 8006 Zürich (CH); Neuner-Jehle, Norbert, 8645 Jona (CH)
(74) Representative: Buntz, Gerhard

(57) **Abstract**

The olfactometer has a sniffing port, a sample supply for generating a sample flow constituted by a carrier gas and sample head space from a saturation chamber and for supplying the sample flow to the sniffing port, a carrier gas supply and mixer for predetermined dilution of the sample flow. The carrier gas supply comprises mass flow controllers with variable flow rates disposed before the sample chamber for providing variable carrier gas flow to the saturation chamber. A multiplicity of capillaries of different diameter connect the mixing means with the sniffing port via individual injectors.

## Description

This invention relates to an olfactometer comprising a sniffing port, sample supply means for generating a sample flow constituted by a carrier gas and sample head space from a saturation chamber and supplying the sample flow to the sniffing port, carrier gas supply means and mixing means for predetermined dilution of the sample flow.

Olfactometers are instruments capable of exactly dosing a portion of the gaseous phase which is present due to the vapor pressure of a sample or analyte in the form of a liquid or a solid, especially an odorant. Generally in all fields olfactometers are desired which cover a large dynamic range with a very high accuracy.

An apparatus for supra threshold olfactometry is known from S.F. Hallowell et al., Cargo Inspection Technologies, SPIE Vol 2276 (1994), 437-448. This publication discloses an apparatus which has been developed at the Auburn University and was used to study and to train dogs for explosives detection. It is based on a fivefold dilution cascade.

A compressor delivers air with a constant pressure which passes a filter and is split in two main channels of which one provides odor-free air used for dilution purposes and the other is used as sample carrier. The sample free air is distributed to five mass flow controllers, which in parallel deliver on five different channels air at a constant flow rate. The sample carrier is passed through mass flow controllers with a variable flow rate. After passing the saturation chamber the carrier gas is split in two new channels, either to reach the sniff-port via passing a valve or to be further diluted by passing again a variable mass flow controller prior to be mixed with sample free air. Five identical dilution cascades are available of which the theoretical dilution limit is 1:1000 per cascade which would enable a dynamic range of 1:10¹². Experimentally shown was a dilution range of 1:100 per cascade (range <10⁸) which only is obtained stepwise.

An important drawback of this system is the obvious path of the sample flow through the mass flow controller units as well as through the valves. The main contamination source of the system is found there. The publication explicitly mentions decontamination procedures. The fact that a mass flow controller is controlled by hot wire detectors, requires calibration data for each gas composition. This makes it a priori impossible to calibrate the system in an exact way. In addition each inaccuracy in a previous cascade can be amplified by the following dilution step according to error evolution laws.

Moreover, this known olfactometer has only one channel and is therefore not capable of performing comparison experiments. Due to the manual operation of the mass flow controllers it is not possible to have a fast and pulse-like stimulus delivery.

The object of the present invention is to provide an olfactometer which is fast, exact, easy to use and adapted for multichannel arrangements and which does not have the disadvantages of the known olfactometers.

This is achieved according to the invention by an olfactometer of the above-described kind which is characterized in that the carrier gas supply means comprises mass flow controlling means with variable flow rate disposed before the sample chamber for providing variable carrier gas flow to the saturation chamber and by a multiplicity of capillaries of different diameter connecting the mixing means with the sniffing port via individual injection means.

The success of a supra threshold olfactometer in applications using odorants depends on its dilution capacity and the sample presentation. A very large continuous dynamic dilution range as well as a perfectly memory-free on/off-switching of the odorous stimulus are the key parameters the present invention has to fulfil these these requirements.

According to a preferred embodiment of the invention the dosage is computer controlled and is available in the form of a self-instructing measurement protocol operating as a virtual instrument which guarantees identical measurement conditions for various sensorial measurements and requires a minimum of training of a panelist.

In the following a preferred embodiment of the invention is described with reference to the accompanying drawings, in which
Fig. 1 shows a schematical diagram of an olfactometer according to the invention,
Fig. 2 shows cross-sectional views of the injection blocks in two different operating positions

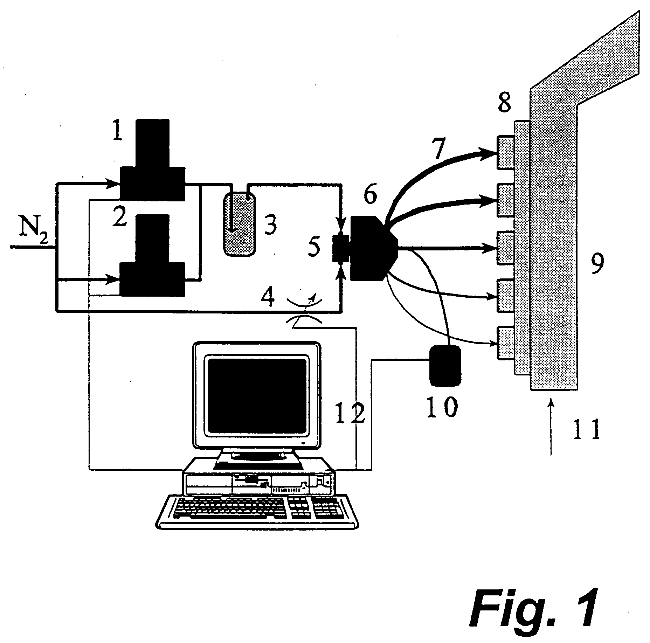

The apparatus shown in Fig. 1 enables performing experiments at a high technical level taking into account important aspects of sensory measurements. It offers a continuously tunable dynamic range of 10% of the saturated headspace concentration down to 10⁻⁸.

The flow of the analyte channel is regulated by two mass flow controllers 1, 2 which are placed before the saturation chamber 3. Mass flow controller 1 is responsible to supply air for higher sample flows and has a range of 50:1000 ml/min whereas mass flow controller 2 has a range of 1:50 ml/min.

From the saturation chamber the sample carrier gas is introduced in the mixing chamber 6 where it can be diluted with nitrogen which is connected as a second gas inlet. The final mixing of the sample carrier gas and nitrogen is achieved in a T-junction followed by a mixing restriction 5. A continuos dilution is achieved from a maximum level of 1:1 down to 1:1000.

The introduced amount of nitrogen is controlled by a valve 4 which is driven by the output signal calculated from a PD (proportional, differential) regulation system which uses the signal from a pressure sensor 10 as input. The pressure is measured in the mixing chamber and monitored by a computer 12. A constant pressure in the mixing chamber is important for a constant flow through the glass capillaries 7. The diameters of these outlets are chosen in such a way, that highly accurate flow rates are available in a range of 1 to 1:10000.

The sample carrier gas is distributed from the mixing chamber to these capillaries which selectively can be injected by a computer controlled injection mechanism 8 into the sniffing port 9 where it is continuously mixed to a gas flow 11 having a constant flow rate of 10 l/min which is approximately the amount of the breathed in air by a human. The measurement program is responsible for selecting the right dilution level and the necessary capillary in order to obtain a continuously accessible dilution range of 1:10'000'000.

In this apparatus contamination sources are reduced to a minimum in a very efficient way. Controlling the flow to the saturation chamber ensures that no mass flow controller and no valve is ever in touch with an odorous molecule.

After fine-machining and honing the stainless steel surfaces of all critical elements are electro-polished (mixing chamber, injection mechanism) giving memory-free measurement conditions. The temperature of the mixing chamber itself can be raised by a few degrees compared to ambient temperature when low volatile compounds have to be measured. A complete cleaning procedure can be done by disassembling the mixing chamber, capillaries and the injection mechanism and by heating them in an oven (similar to GC-column purification).

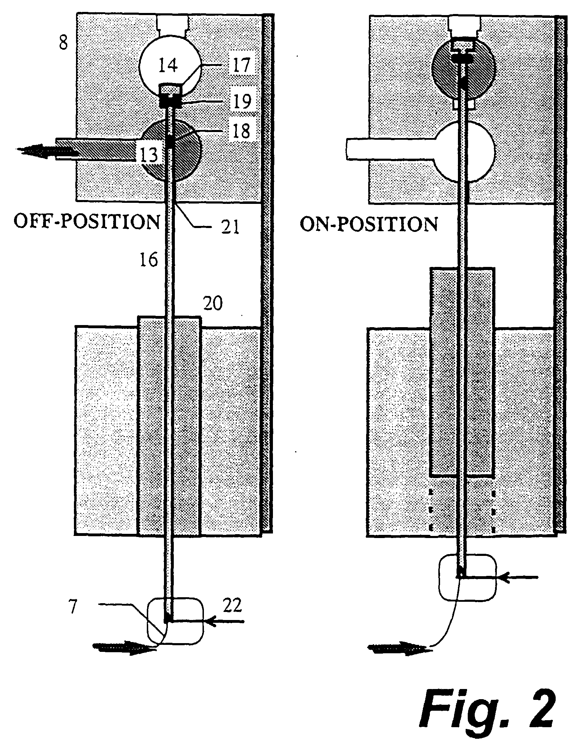

The final dilution step is done as previously mentioned, by injecting the sample flow from a capillary into the transport channel 13 of the sniff port by means of an injection block whose cross section is shown in Fig. 2. The injection block 8 is constituted by a cube (200 x 25 x 20 mm) having two longitudinally machined holes, i.e. an exhaust line 13 and a transport line 14 to the sniff port 9. The block contains 6 individual injection systems for the glass capillaries. The two longitudinal holes are drilled in the horizontal center of the block but are vertically connected through a small guiding holes which are machined along the vertical axis.

Adapter capillaries 16 are capped 17 but have outlets 18 a few mm below the top. Between the top and sample inlets O-ring seals 19 are inserted in order to prevent the sample stream going into the transport channel while the adapter-capillaries are held in the off-position shown in Fig. 2a where the capillary outlet is flushed directly into the exhaust line. Holes 21 are sealed with teflon. The adapter-capillaries 16 in fact are used for mechanical protection of the glass capillaries 7 which are delicate and constitute a well-calibrated system.

A computer-controlled pneumatic compact-cylinder 20 switches each adapter capillary from the off- to the on-position Fig. 2b. Introducing an adjusted make up gas flow (nitrogen) from the lower end 22 of the adapter-capillary 16 makes sure that even for the lowest flow rates measured at the smallest glass-capillary the sample reaches the transport line 14 in the same time as measured on the larger glass-capillaries.

A flush-line 23 constantly purges the O-ring-seal 19 against the flow-direction of the sample carrier gas which ensures that no traces of the sample can reach the transport line 14. An optimum "blank" is achieved when all injection units are switched to the "off"-position. Intensity rating or ranking is well known in sensory research and several measurement methods have been developed. Magnitude estimation, reference scaling and category scaling are the most popular ones. Recently the labelled magnitude scale has been presented and compared to magnitude estimation.

All the operations on the olfactometer are computer controlled and the instrument control has been developed under LabVIEW. So called virtual instruments (VI's) are programmed and displayed on the screen if necessary. On such a VI the operater can set for example a desired concentration which is then delivered from the olfactometer. The flexibility of the software allows to summarize any kind of measurement sequence as a measurement protocol.

A very fast and universal method of measuring the dose/response behavior of odorous substances has been successfully applied using the LMS. The measured and fitted values can be mached to the ASTM values and any other substance can be compared to the standard. An evaluation of linalool for instance shows visually an increased slope of the dose/response curve.

Intensity matching expenments can be carried out simultaneously on two modules. This kind of measurement allows the determination of the just notable differences at any concentration level in the supra-threshold regime of an odorant.

Adaptation phenomena can be measured in a separate measurement protocol. The measurement protocol uses the following sequence:
first sequence according to dose/response curve measurement using LMS
second sequence is the same but prior to the evaluation of the delivered sample the panellists nose is exposed 5 seconds to a very high dose of the same odorant.

The supra-threshold olfactometer according to the present invention has a flexible design which allows to attach different sample saturation chambers. It is possible to perform intensity measurements on applied systems such as fragrances deposited on laundry etc. These measurements allow a fast comparison of substantivity of different products.

## Claims

1. An olfactometer comprising a sniffing port, sample supply means for generating a sample flow constituted by a carrier gas and sample head space from a saturation chamber and supplying the sample flow to the sniffing port, carrier gas supply means and mixing means for predetermined dilution of the sample flow characterized in that the carrier gas supply means comprises mass flow controlling means with variable flow rate disposed before the sample chamber for providing variable carrier gas flow to the saturation chamber and by a multiplicity of capillaries of different diameter connecting the mixing means with the sniffing port via individual injection means.

2. An olfactometer according to claim 1 characterized by computer control means for controlling the dosages.
